Europäisches Patentamt

(19) European Patent Office

Office européen des brevets

(11) Publication number: **0 068 691**
A2

# (12) EUROPEAN PATENT APPLICATION

(21) Application number: 82303035.8

(22) Date of filing: 11.06.82

(51) Int. Cl.³: **C 12 N 15/00,** C 12 N 9/52, C 12 N 9/60, C 12 N 1/00, C 07 H 21/04, C 12 Q 1/38 // C12R1/19, C12R1/865, C12N9/64

(30) Priority: 17.06.81 GB 8118688
11.11.81 GB 8133998
01.12.81 GB 8136185
10.02.82 GB 8203907

(43) Date of publication of application: 05.01.83 Bulletin 83/1

(84) Designated Contracting States: DE FR GB IT NL SE

(71) Applicant: CELLTECH LIMITED, 244-250 Bath Road, Slough SL1 4DY Berkshire (GB)

(72) Inventor: Carey, Norman Henry, Russels Close High Street, Chinnor Oxon (GB)
Inventor: Doel, Michael Terence, Woodbury Wycome Road, Studley Green Bucks (GB)
Inventor: Harris, Timothy John Roy, 54 Red Rose Binfield, Bracknell Berks (GB)
Inventor: Lowe, Peter Anthony, 9 Melrose Avenue, Reading Berks (GB)
Inventor: Emtage, John Spencer, 12 Benjamin House Amersham Hill, High Wycombe Bucks (GB)

(74) Representative: Votier, Sidney David et al, CARPMAELS & RANSFORD 43, Bloomsbury Square, London WC1A 2RA (GB)

(54) A process for the production of a polypeptide.

(57) A process is described for the production of the proteolytic enzyme chymosin. The process involves cleaving chymosin precursor polypeptides such as methionine-prochymosin, preprochymosin and methionine-chymosin expressed from a host organism which has been transformed with a vector system carrying the relevant gene. The gene sequence of preprochymosin is given. The vector systems used are plasmids adapted for the transformation of either bacterial or eukaryotic host organisms. E. Coli and saccharomyces cerevisiae are specific examples of suitable host organisms. There is also described an oligonucleotide capable of being used as a specific hybridisation probe or transcription primer for mRNA containing the nucleotide sequence coding for chymosin. A microassay for chymosin activity is also described.

EP 0 068 691 A2

# PROCESS FOR THE PRODUCTION OF A POLYPEPTIDE

## Field of the Invention

This invention relates to the field of recombinant DNA biotechnology. More particularly it relates to a process for the manufacture from readily-available materials of a microorganism capable of producing chymosin, a milk clotting enzyme; prochymosin, the zymogen of chymosin, which may be readily converted into chymosin, and preprochymosin which is a precursor of prochymosin.

## Background of the Invention

Milk consists essentially of a colloidal suspension of the protein casein. It has been known for many years that an extract from the lining of the fourth stomach of the calf (abomasum) has the effect of destabilising this colloid. The resulting agglutination of the casein molecules produces an effect which is commonly known as curdling. This curdling is the primary stage in the process of cheese making.

During the first few weeks of the calf's life, until it is weaned, it suckles cows milk. The digestion of this milk is assisted by the presence of acid proteinases (collectively known as rennin or rennet) in the fourth stomach of the calf. Rennin has a proteolytic action on kappa casein in milk which results in a cleavage of the protein chain. The resulting protein (para kappa casein) is unable to stabilise in a colloid and curdling occurs.

For many years the only source of rennin for the cheese making industry was the stomach of calves. Recently however there has been a growth of the cheese making industry which has resulted in the demand for rennin outstripping the supply from natural sources. The economics of breeding calves for slaughter within 12 weeks of birth are not in favour of large scale production of

0068691

natural rennin. Finding alternatives has not however been easy.

In attempting to find a replacement for natural rennin an acid proteinase, or a mixture of acid proteinases with very low proteolytic activity, is required. These conditions must be met in order to obtain a satisfactory yield of curds and in order to produce a satisfactory maturation of the cheese. A proteolytic enzyme of greater activity would tend to disrupt the casein protein to such an extent that it would dissolve during production and ripening of the cheese. This is undesirable. A particular disadvantage is that smaller segments of the casein protein may afford the final product a bitter taste that is often associated in the consumer's mind with an "off" flavour.

Two alternative milk curdling agents have found acceptance in the cheese making industry.

The first of these is derived from animal enzymes. They comprise blends of calf rennin with bovine pepsin. (Bovine pepsin is an enzyme found in the stomach of mature cattle and is the proteolytic enzyme which replaces the rennin found in unweaned calves.) Such compositions of naturally occurring proteolytic enzymes have found wide acceptance as an alternative to pure calf rennin.

Secondly, and perhaps more importantly, a range of proteolytic enzymes from fungi have been developed. These enzymes, of microbial origin,-were developed in Japan some years ago. The three major sources of microbial milk curdling agents are the following fungal strains:

mucor pusillous

mucor miehei, and

endothia parasitica.

The disadvantage of milk curdling agents produced from these fungal strains is that in general they have considerable proteolytic effect. As mentioned above, this reduces the yield of curd and therefore the yield of cheese. Bitter flavours may also result. These factors are particularly noticeable in cheeses which require long maturation. For this reason microbial milk curdling agents are only really suitable for cheeses which require a short maturation.

The main proteolytic enzyme found in rennin is chymosin. This protein is produced from an inactive precursor called prochymosin which is secreted by the calf fourth stomach (abomasum). Chymosin has been the subject of considerable research in recent years. One result of this is that the complete amino acid sequence of the zymogen prochymosin has been determined. (See Foltmann et al Proc. Nat. Acad. Sci. USA 77 2321-2334 (1977) and J. Biol. Chem. 254 8447-8456 (1979). The first of these papers presents the complete 365 amino acid sequence of prochymosin. The second paper gives the sequence of the single peptide chain of 323 amino acid residues; this being the primary structure of chymosin. The removal of the 42 amino acid residues from the amino terminal of the prochymosin chain results in the activation of the enzyme. We have discovered that the primary translation product of the calf stomach prochymosin gene is a precursor preprochymosin having 16 amino acids N-terminal to the prochymosin moiety. The present invention involves a process for the production of calf stomach chymosin, prochymosin and preprochymosin from a bacterium utilising the technique of recombinant DNA or genetic manipulation. The prochymosin may then be cleaved by an autocatalytic, pH-dependent, reaction.

Brief Description of the Invention

According to an aspect of the present invention we provide a process for the production of chymosin comprising the step of cleaving methionine-chymosin, methionine-prochymosin or preprochymosin expressed by a host organism transformed with a vector system carrying a gene coding for methionine-chymosin, methionine prochymosin or preprochymosin respectively.

In a further aspect we provide a process for the production of chymosin comprising the steps of

a) inserting a gene coding for methionine-prochymosin into a vector system

b) transforming a host organism with the gene carrying vector system

c) cleaving methionine-prochymosin expressed by the host organism to form chymosin.

In a further aspect of the invention we provide a process for the production of methionine-prochymosin comprising the steps of

a) inserting a gene coding for methionine-prochymosin into a vector system

b) transforming a host organism with the gene carrying vector system

c) isolating methionine-prochymosin expressed by the transformed host organism.

In a further aspect of the invention we provide a process for the production of preprochymosin comprising the steps of

a) inserting a gene coding for preprochymosin into a vector system

b) transforming a host organism with the gene carrying vector system

c) culturing the host organism to permit expression. Preferably in this aspect preprochymosin expressed by the transformed host organism is isolated.

In a further aspect of the invention we provide a process for the production of methionine-chymosin comprising the steps of

a) inserting a gene coding for methionine-chymosin into a vector system

b) transforming a host organism with the gene carrying vector system

c) isolating methionine-chymosin expressed by the transformed host organism.

In a further aspect of the invention we provide a process for the production of prochymosin comprising the steps of

a) inserting a gene coding for preprochymosin into a vector system

b) transforming a host organism with the gene carrying vector system

c) cleaving preprochymosin expressed by the transformed host organism to form prochymosin.

In a further aspect of the invention we provide a process for the production of chymosin comprising the steps of

a) inserting a gene coding for preprochymosin into a vector system

b) transforming a host organism with the gene carrying vector system

c) cleaving preprochymosin expressed by the transformed host organism to form chymosin.

In another aspect of the invention we provide the polypeptides methionine-prochymosin, methionine-chymosin and preprochymosin. Preferably they are produced by the methods of the aspects of the invention described above. (We also provide chymosin produced by those methods).

In another aspect of the invention we provide the genes coding for methionine-prochymosin, methionine-chymosin and preprochymosin.

In another aspect of the invention we provide a system including a gene coding for methionine-prochymosin, preprochymosin or methionine-chymosin. The vector system may include a controllable expression promoter sequence. In one preferred embodiment the vector system is capable of transforming a bacterial host organism and including one or more sequences capable of conferring a phenotype upon a transformed host organism different from that of the untransformed host organism. Preferably the vector system includes the sequence for the E. Coli tryptophan operon promoter. In a second preferred embodiment the vector system is capable of transforming a eukaryotic host organism and including one or more sequences capable of
conferring a phenotype upon a transformed host organism
different from that of the untransformed host organism.
Preferably the host organism is a yeast. Further preferred is a vector system derived from the bacterial plasmid
pBR322 and the yeast 2u plasmid. Further preferred is a
vector system including yeast phosphoglycerate kinase
(PGK) gene promoter sequence.

In another aspect of the invention we provide a host
organism transformed by a vector system as described above.
Preferably the host organism is a bacterium and is suitably
Escherichia Coli for example of the strain HB101 or RV308.

In the alternative the host organism may be a/suitably yeast
saccharomyces cerevisiae.

In another aspect of the invention we provide an
oligonucleotide including the nucleotide sequence;

    5'    GTT.CAT.CAT.GTT    3'

wherein G is guanine

    T is thymine

    A is adenine  and

    C is cytosine.

Preferably the oligonucleotide is used in a process
for the detection of mRNA containing the nucleotide
sequence coding for a polypeptide including the amino
sequence of chymosin                                as a
hybridisation probe. In the alternative the oligonucleotide is used in a process for the preparation of a portion
of single stranded cDNA coding for a polypeptide including
the amino acid sequence of chymosin as a transcription
primer.

In a final aspect of the invention we provide an
assay for chymosin activity wherein one or more dilutions
of a sample are incubated with milk in microtitre plate
wells. Preferably the assay comprises the steps of

    a) preparing series dilutions of a sample

    b) adding a standard sample of milk to each dilution

    c) incubating for a fixed period of time

d) observing presence or absence of milk sample
   clotting.

Preferably the milk is reconstituted dried skimmed
milk.

Brief Description of the Drawings

In the following description various embodiments of the present invention are described with reference to the accompanying drawings in which:

Figure 1 - is a schematic diagram showing the construction of recombinant chymosin, prochymosin and preprochymosin plasmid vectors

Figure 2 - shows the nucleotide sequence of cDNA obtained by transcription of mRNA using a synthetic oligonucleotide specific primer

Figure 3 - a) shows restriction enzyme maps of some of the clones attained

b) shows the restriction enzyme map of clone 118.

Figure 4 - shows the complete nucleotide sequence of preprochymosin mRNA (including within it the sequences of prochymosin mRNA and chymosin mRNA).

Figure 5 - shows restriction maps of the plasmid vectors constructed for the expression of met-chymosin, met-prochymosin and preprochymosin in E. Coli.

Figure 6 - is a schematic diagram of the plasmid vectors used for the expression of met-chymosin, met-prochymosin and preprochymosin in yeast.

Figure 7 - shows the DNA sequences around the Bam HI insertion site in the plasmids used for expression in yeast.

Figure 8 - shows a Tris-SDS gel of total E. Coli proteins produced by pCT70 in HB101 following a shake flask fermentation.

Figure 9 - shows a Tris-SDS gel of total E. Coli proteins produced by pCT70 following fermentation in a 10L fermenter.

Figure 10 - a) shows an isoelectric focusing/Tris-SDS gel of total $^{35}$S-methionine labelled E. Coli proteins from pCT70

b) shows an isoelectric focusing/Tris-SDS gel of total $^{35}$S-methionine labelled E. Coli protein from pCT54.

Figure 11 - shows a Tris-SDS gel of an experiment involving the precipitation of prochymosin produced by pCT70 in E. Coli by anti authentic calf prochymosin polyclonal antisera.

Figure 12 - shows a Tris-SDS gel of various fractions in the early stages of purification of prochymosin produced by pCT70 in E. Coli.

Figure 13 - shows the separation of authentic calf chymosin from material present in crude acidified extracts of E. Coli by gel permeation chromatography on Ultrogel AcA54.

## Description of the Embodiments

In order to describe the invention fully we first describe the process in general and then give details of a typical preparation.

The first stage in the manufacture of a microorganism, such as is required here, is the isolation of the gene which codes for the required protein. In the case of prochymosin the size of the protein and the redundancies in the genetic code make the chemical synthesis of such a gene unattractive. One approach to a clonable gene is to prepare prochymosin mRNA from the mucous membranes of the calf's stomach (see Uchiyama et al. Agric. Biol. Chem 44 1373-1381 (1980).

The mRNA isolation may be made by one of several methods, for example total RNA (Kirby Methods in Enzymology Vol. XII Academic Press (1968) or polysomal RNA extraction (Palmiter J. Biol. Chem. 248 2095-2106 (1973). The concentration of prochymosin mRNA may be increased by

0068691

further stages of purification for example oligo(dT) cellulose column chromatography or sucrose gradient centrifugation. When a reasonably pure sample of prochymosin mRNA has been produced the mRNA preparation may then be transcribed with AMV reverse transcriptase (see Emtage et al. Nucl. Acid Res. 6 1221-1240 (1979)). This results in single stranded prochymosin cDNA. This copy may be isolated and re-transcribed with either AMV reverse transcriptase or DNA polymerase I (see Estratiadis et al. (1976) Cell. 7 279-288). Both processes produce a double stranded full length gene with the two strands of the gene linked together by a hairpin loop at one end. This loop may be severed by S1 endonuclease (see Vogt (1973) Eur. J. Biochem. 33 192-200). Since the use of S1 nuclease leads to the loss of some nucleotide sequence from the end of the synthesised gene an alternative method may be used. In this method a short tract of oligodeoxycytidine is attached to the 3' end of the first strand of cDNA using the enzyme terminal transferase (Land et al (1981) Nucl. Acid Res. 9 2251). A short primer of oligodeoxyguanosine may then be used to prime second strand cDNA synthesis. In this procedure complete nucleotide sequences are available for cloning. A schematic representation of the steps of cDNA synthesis is given in Figure 1. (In this Figure the broken lines represent mRNA and the solid lines represent cDNA).

The resulting full length prochymosin gene is now prepared for insertion into a suitable plasmid vector, for example by ligation of a synthetic linker to the prochymosin gene (Porter et al. (1979) Nature 282 471-477). This linking may be achieved using, for example, T4 DNA ligase. The linkers may then be cut with a suitable restriction enzyme to produce staggered cohesive ends on the prochymosin DNA. These cohesive ends facilitate its attachment to the plasmid vector. Alternatively the prochymosin DNA may have short tracts of oligodeoxycytidine attached to its ends and these may then be hybridised to complementary tracts of oligodeoxyguanosine attached to the ends of the plasmid vector (MacGillivray et al. (1980) Proc. Nat. Acad. Sci. USA 77 5153-5157). Once a suitable vector has been selected a staggered incision is made into its ring structure by use of a suitable restriction enzyme and the prochymosin DNA section is ligated into the ring. The vector used initially may not have the capacity to express isolated genes. The vector selected for expression must contain a strong promoter to allow the expression of the inserted gene, and must also contain the usual identifying genes coding for resistance to antibodies etc. The recombinant plasmids so produced are used to transform a suitable host organism (for example Escherichia coli) (Cohen et al. (1972) Proc. Nat. Acad. Sci. 69 2110-2114).

The prochymosin mRNA used in the initial stages of producing this host organism is not completely pure and therefore the cDNA which is cloned consists of a population of species only some of which carry DNA sequences specifying prochymosin. A screening step is therefore essential to identify which plasmids carry the DNA sequence of prochymosin. This suitably involves hybridisation analysis with a synthetic oligonucleotide having a sequence specific for part of the amino acid sequence of prochymosin (see Grunstein and Hogness (1975) Proc. Nat. Aca. Sci. 72 3961-3965 and Foltmann et al. (1977) Proc. Nat. Acad. Sci. 74 2321-2334). Using this process it is possible to detect prochymosin specific clones from the library of clones produced by the process up to this point. From clones specific to prochymosin production a full length cDNA copy may be selected by restriction enzyme digestion of the plasmid DNA followed by size fractionation using polyacrylamide gel electrophoresis. (Doel et al (1980) Nucl. Acid. res. 8 4575-4592). The selected plasmid is then transferred into the chosen expression vector taking care to produce the correct orientation and phasing of the insert with respect to the plasmid control sequences. When full size complementary DNA gene, in the correct orientation, is obtained the expression of the gene will be achieved by the normal processes of host cell metabolism and the production of prochymosin may be monitored by techniques of protein chemistry known to the art. We have devised procedures for the isolation and purification of prochymosin from microbial cells involving the exposure of the proteins to acid pH. It is known that prochymosin can be converted to active chymosin by an autocatalytic process involving exposure to acid pH (Asato and Rand (1977) Biochem. J. 167 429-434). This activation results from the removal of 42 amino acids from the amino terminal of prochymosin (Pedersen et al. (1979) Eur. J. Biochem. 94 573-580).

A more detailed account of each of the individual steps in the process will now be given.

In a specific example, the preparation of RNA from the calf stomach was performed as follows.

The fourth stomach (abomasum) for a freshly slaughtered unweaned calf (10 days) was opened and rinsed with an isotonic buffer. The reddish mucosal layer was scraped off with a scalpel and kept at 0°C. The tissue was either used immediately in an RNA preparation or frozen in liquid nitrogen prior to storage at -80°C. About 50 gm of mucosa was obtained per stomach.

RNA was isolated from the abomasal mucosa by the following method, essentially that of Kirby (Methods in Enzymology, Vol XII. Academic Press (1968) p.87). The scraped abomasal mucosa were chopped finely with scissors before being homogenised. 25 gm of tissue was mixed with 45 ml of 1% sodium chloride (w/v), 45 ml of 6% sodium 4-amino salicylate and 45 ml of phenol : cresol mixture (Phenol - 500 gm, m-cresol - 70 ml, water - 55 ml, 8-hydroxyquinoline - 0.5 gm.. The mixture was stirred at room temperature for 20 mins, before centrifuging at 6000 xg for 30 mins at 5°C. The upper phase was removed and adjusted to 3% (w/v) with sodium chloride. This layer was then extracted with half its volume of the

phenol : m-cresol mixture for '. -irs at room temperature and then centrifuged at 8000 xg for 15 mins at 50°C.  The aqueous phase was removed and mixed with 2 volumes of ethanol, pre-cooled at -20°C.  The mixture was kept for 20 mins at 0° and the precipitated RNA and DNA centrifuged off at 8000 xg for 10 mins at 5°.  The nucleic acid pellet was gently resuspended in 100 ml of 2% (w/v) sodium acetate, pH 6.0 and the solution adjusted to 3 $\underline{M}$ in sodium chloride by addition of solid.  It was kept overnight at 4° and the precipitate of RNA centrifuged at 8000 xg for 15 mins at 5°.  The pellet was extensively washed with 3 $\underline{M}$ sodium acetate, pH 6.0 to remove DNA and soluble RNA, then with 70% (v/v) ethanol to remove sodium acetate.  The pellet was then dried and redissolved in a dilute buffer solution and stored at -20°C.  Yield was about 30-40 mg of RNA.

This extract of calf's stomach RNA was then further purified by the following chromatographic process.  It would also be possible to use, for example, sucrose gradient centrifugation.

15 mg of calf stomach RNA was adjusted to about 1 mg/ml in a buffer containing 0.5 $\underline{M}$ sodium chloride, 20 mM HEPES pH 7.5, 2mM EDTA and 0.1% SDS (loading buffer).  This solution was applied to a small chromatographic column containing 2 gm of oligo (dT) cellulose (B.R.L. Ltd. Bethesda, Md) which had been extensively washed with loading buffer.  After loading onto the column under gravity any unbound RNA was washed off with loading buffer until the optical density of the elute dropped below 0.05 at 260 nm.  The bound mRNA fraction was then eluted with several column volumes of 20 mM HEPES pH 7.5, 1mM EDTA, 0.1% SDS.  The mRNA was recovered by precipitation at -20° with 2 volumes of absolute ethanol and the precipitate centrifuged at 15,000 xg for 10 mins at 4°.  300-400 microgrammes of mRNA fraction were obtained.

The next stage in the process is the preparation of single stranded cDNA from the prochymosin mRNA.  In order to produce a full length cDNA single strand from a mRNA single strand it is necessary to prime the mRNA at a fixed site on the mRNA must be established from which transcription of the cDNA may begin.

In this work two primers have been used. The first of these was oligo(dT) $_{12-18}$. This primer comprises a series of thymidine (T) residues. A characteristic of almost all eukaryotic mRNA is that it includes at one end of the chain a series of adenine (A) residues. The above-mentioned primer can hybridise to this oligo (A) grouping and form a start point for the transcription of a single strand cDNA. The method followed was essentially that of Emtage et al. (Nucl. Acid. Res. 6 1221-1240, (1979). The mixture contained 50 mM Tris. Cl pH 8.3, 10 mM magnesium chloride, 0.5 mM dCTP labelled with $^{32}$P, 5 mM DTT, 0.01% Triton X-100, 50 ug/ml of actinomycin D, 50 mM potassium chloride, 10 ug/ml oligo(dT), 20-50 ug/ml of mRNA fraction, 10-60 U/ml of AMV reverse transcriptase (IUB Cat. No. E.C.2.7.7.7.). After incubation at 37°C for 90 mins the reaction was terminated by adjusting the solution to 0.2% SDS and 20 mM EDTA. The solution was extracted with an equal volume of 1:1 (v/v) phenol : chloroform and the aqueous layer concentrated by precipitation with 2 volumes of absolute ethanol at - 20°C. The recovered cDNA was dissolved in water, adjusted to 0.1M with sodium hydroxide and incubated at 60°C for 30 mins. After neutralisation with acetic acid the material was chromatographed on a Sephadex G-150 column in a buffer containing 50 mM sodium chloride and 0.1% SDS. Labelled material eluting in the void volume of the column was pooled and precipitated with 2 volumes of ethanol. The size of the cDNA was determined by electrophoresis against standard DNA fragments in a 5% acrylamide gel using a buffer containing 90 mM tris, 90 mM boric acid, 2.5 mM EDTA, pH 8.3 $^{32}$P - labelled cDNA was located by autoradiography of the gel using Fuji-RX film. cDNA having a size of greater than 800 bases was cut and eluted from the gel by electroelution into a buffer containing 0.5 M ammonium acetate, 0.01M magnesium acetate, 0.1% SDS and 0.1 mM EDTA. This high molecular weight cDNA was used for the synthesis of double stranded material.

One disadvantage of this method of cDNA transcription is that it will produce cDNA from all species present in the mRNA population that is isolated ie the cDNA will be heterogeneous with respect to its sequence. One way of overcoming this is to use, instead of oligo $(dT)_{12-18}$, a sequence specific primer.

Another drawback in the use of oligo$(dT)_{12-18}$ primed cDNA is that transcription tends to stop short of the 5' end of the mRNA. This may be due to specific secondary structure features or to generally suboptimal conditions in the enzyme reactions. The absence of sequences derived from the 5' end of the mRNA is a severe drawback when expression of the cloned sequence is a primary aim. To obtain specifically 5' end sequences in cDNA a primer can be used which hybridises to the mRNA in the middle or near the 5' end.

In order to do either of these things it is necessary to locate a sequence of amino acids in the prochymosin protein for which a low degeneracy in the genetic code exists. That is to say the number of nucleotide sequences coding for that particular series of amino acids is low enough to make it viable to synthesise all the possible combinations. We have noted the presence of the following amino acid sequences in prochymosin.

<u>182</u>                              <u>189</u>

asp - asn - met - asn - arg

The messenger RNA for this sequence of amino acids may be predicted:-

```
5'                                    (A)   3'
   (T) (T)          (T)  (C)   (C)
GA .AA .ATG.ATG.AA     .    G
   (C) (C)          (C)  (A)   (T)
                              (G)
```

The centre four codons of this sequence taken together
have only four-fold degeneracy. A set of oligonucleotides
complementary to the possible mRNA sequence can be design-
ed:-

5                              3'

    A TT.CAT.CAT. A TT
    A TT.CAT.CAT. G TT
    G TT.CAT.CAT. G TT
    G TT.CAT.CAT. A TT

One of these four sequences must exactly match the natural
mRNA sequence and will therefore hybridise to that area
of the prochymosin mRNA.   An alternative set of oligonucleotides for the same region would be:

5'

    TT.CAT.CAT. A TT. A TC
    TT.CAT.CAT. A TT. G TC
    TT.CAT.CAT. G TT. G TC
    TT.CAT.CAT. G TT. A TC

These oligonucleotides were synthesised using conventional
phosphotriester synthetic methods (Hsiung et al. (1979)
Nucl. Acid. Res. 6 1371-1385) and were purified by high
pressure liquid chromatography on an ultrasil column
(Altex Limited USA).   These synthetic oligonucleotides
were used as primers for the transcription of single
stranded cDNA from prochymosin mRNA.

2 ug of each oligonucleotide was labelled with $^{32}$P
phosphate at its 5' end using T4 polynucleotide kinase
and $-^{32}$P-ATP.

The mixture contained 0.1-1.0 mCi of $-^{32}$P-ATP,
50 mM tris. Cl pH 7.8, 5 mM magnesium chloride, 10 mM
β-mercaptoethanol and 5-20 units of T4 polynucleotide
kinase.   After incubation for 30-60 minutes at 37° the
solution was extracted with an equal volume of 1:1 (v/v)
phenol: chloroform and the aqueous layer passed down a
Sephadex G-50 column equilibrated with 50 mM sodium
chloride, 20 mM Tris. Cl pH 7.5, 0.1% SDS.   The excluded
fractions containing the labelled oligonucleotides, were pooled and precipitated by the addition of 2 volumes of absolute ethanol. The precipitated material was recovered by centrifugation at 15,000 xg for 15 minutes at 4°.

The four labelled oligonucleotides were then used as primers of cDNA synthesis as described above for oligo(dT) 12-18 above except that no $^{32}$P-dCTP was included in the reaction mixture.

It was found that only one of the four oligonucleotides tested, that with the sequence 5'-GTT.CAT.CAT.GTT-3', was able to prime cDNA synthesis on the calf stomach mRNA population. This defines the mRNA sequence in the region of the methionine doublet as being that complementary to the sequence of this particular oligonucleotide.

The cDNA produced was maximally about 650 bases long as measured by gel electrophoresis which is in agreement with previous estimates of the distance between the met-met sequence and the 5' end of the mRNA. It consisted however of several discrete molecular weight species. This cDNA is single stranded and labelled at its 5' end with a single $^{32}$P-phosphate. These are the requirements for sequencing a length of DNA by the chemical method of Maxam and Gilbert (Proc. Natl. Acad. Sci. USA 74 560 (1977)). The primed cDNA was therefore sequenced by these methods and a DNA sequence derived which is consistent with the previously known amino acid sequence of the region of prochymosin protein from which the primer nucleotide sequence was derived. The sequencing methods are now standard in many laboratories, a step by step method is given in Methods in Enzymology 68 499-560 (1980). The sequence derived is set out in Figure 2, together with the relevant stretch of amino acid sequence and the restriction enzyme recognition sites which it contains. Especially interesting is the presence of recognition sites for the enzymes, Sma I, Eco.RI, Hind III and Bam HI. There are no Sma I sites in the plasmid pAT153 used for the initial cloning and the other three are also rare

sites. One or other of these provides a useful site for joining the specific 5' cDNA produced from the primer oligonucleotide to the short cDNA produced from oligo(dT) 12-18 priming.

The fact that the cDNA obtained using the oligonucleotide 5'-GTT.CAT.CAT.TGG-3' is heterogeneous in size and yet can be used unfractionated in the chemical sequencing reactions to give a readable sequence indicates that it does in fact hybridise only to one specific point on one specific mRNA. This means that cDNA made using the oligonucleotide can be used as a totally specific hybridisation probe in the screening of bacterial colonies obtained in the cloning experiments (see screening procedures below). This extended primer cDNA forms more stable interactions with the plasmid DNA than do the short oligonucleotides themselves and so the cDNA is preferred as a probe.

The above methods of transcription produce a DNA molecule with a DNA "hairpin" structure at its 3' end. This may be used in producing the second strand of the DNA. Two methods may be used, either the single strand of the DNA. Two methods may be used, either the single strand cDNA may be subjected to, for example, E. Coli DNA polymerase I, or to AMV reverse transcriptase which, whilst it would under normal circumstances produce a copy of single stranded DNA, will in this case produce a double stranded cDNA since the hairpin structure will act as a primer. In the preparation presently being described the single stranded cDNA was converted into a double stranded form by incubation in a mixture containing 50 mM Tris. Cl pH 8.3, 20 mM DTT, 10 mM magnesium chloride, 0.5 mM dGTP, 0.5 mM dATP, 0.5 mM dTTP, 0.5 mM dCTP, 2-10 µg/ml of AMV reverse transcriptase. After incubation at 45°C for 4 hours the solution was adjusted to 0.2% SDS and 20 mM EDTA and extracted with an equal volume of 1:1 (v/v) phenol:chloroform. The aqueous layer was chromatographed on a Sephadex G-150 column as described above, the fractions eluting at the void volume were pooled and pre-

cipitated with 2 volumes of absolute ethanol. The size of the synthesised DNA was determined by gel electrophoresis against standard DNA fragments on a 5% acrylamide gel using a buffer containing 90 mM Tris, 90 mM boric acid and 2.5 mM EDTA, pH 8.3. Labelled DNA was located by autoradiography of the gel using Fuji-RX film. Double stranded cDNA with a size greater than 800 bases was cut out and eluted from the gel as described for single stranded material above. This high molecular weight material was used for cloning experiments.

The hairpin loop at one end of the cDNA was removed by digestion with $S_1$ nuclease, which is prepared from $\alpha$-amylase (Vogt. V M. Eur. J. Biochem $\underline{33}$ 192-220 (1973)). The digestions were carried out in a solution containing 150 mM sodium chloride, 25 mM sodium acetate pH 4.6, 1 mM zinc sulphate, 10-100 μg/ml of cDNA and 25-50 units/ml of $S_1$.

Any residual "raggedness" at the ends of the double stranded cDNA is repaired by incubating the cDNA with DNA polymerase I (the so-called "Klenow fragment") in a 20 μl mixture containing 50 mM Tris. Cl pH 7.5, 5 mM $MgCl_2$, 2mM mercaptoethanol, 0.5 mM of each of the four deoxyribonucleotide triphosphates and 2 units of DNA polymerase I (Klenow fragment). Incubation was for 15 minutes at 15°C. To ensure retention of nucleotide sequences at the end of the cDNA gene an alternative method modified from the procedure of (Land et al. (1981) Nucl. Acid. Res. $\underline{9}$ 2251) was used. Single stranded cDNA prepared as described above was extended at its 3' end with oligodeoxycytidine in a reaction containing 100 mM sodium cacodylate, pH 7.1, 0.1 mM DTT, 10 mM cold dCTP, 1 mM $CoCl_2$, 250 μCi/ml of $^3$H-dCTP and 10 μl/ml of terminal transferase. The reaction was monitored by measuring the incorporation of labelled dCTP and terminated by phenol extraction when an average of 50-100 residues of deoxycytidine had been added to each molecule. Second strand synthesis on this extended cDNA was accomplished by annealing oligodeoxy- guanosine (12-18 residues long) to it in a solution containing 10 µg/ml of cDNA 50 mM Tris. Cl pH 8.2, 30 mM KCl, 10 mM MgCl$_2$ and 50 ug/ml of oligo dG$_{(12-18)}$. The mixture was incubated for 30 minutes at 68° then cooled to 37° over 60 minutes before adjusting it to contain 10 mM DTT, 0.4 mM of each of the four deoxynucleoside triphosphates and 400 units/ml AMV reverse transcriptase. The mixture was incubated at 38° for 10 minutes then at 42°C for 3 hours before phenol extraction and size selection as described above.

A preferred method for introducing the double cDNA into a plasmid involves the use of homopolymeric oligonucleotide "tails" attached to cDNA and plasmid (Otsuka (1981) Gene 13 339-346). After incubation with the DNA polymerase I (Klenow fragment) to neaten the ends, the cDNA was tailed with oligodeoxycytidine using terminal transferase from calf thymus (EC 2.7.7.31) in the presence of 0.5 mM cobalt chloride and 0.1-1 mM $^3$H-dCTP. DNA of the plasmid pAT 153 which had been cut with Pst I was then tailed with oligodeoxy-guanosine using terminal transferase in the presence of 10 mM magnesium chloride and $^3$H-dGTP. The reactions were monitored by measuring the incorporation of radioactivity into trichloroacetic acid precipitable material and the reactions terminated when incorporation showed that an average of 30-40 residues had been added to each 3' end. The mixture was deproteinised with phenol and chloroform and the tailed DNAs recovered by ethanol precipitation.

Equimolar amounts of the two tailed species were annealed together in 50 µl of a mixture containing 0.15 M sodium chloride, 0.01 M Tris. Cl pH 7.4, 1 mM EDTA by heating at 68° for 30 minutes and allowing the bath to cool to room temperature over about 4 hours. The annealed DNA was used to transform competent bacterial cells as described below.

From the population of transformed organisms selection is made (possibly based on sensitivity to to tetracycline or ampicillin), to determine which of the organisms have successfully incorporated recombinant DNA.

In the present example E. coli HB101 (Boyer H.W. and Roulland Dussoix D. (1969) J. Mol. Biol. 41 459-472) was transformed with pAT 153 carrying the synthetic genes using the method of Katz et al. (1972 J. Bacteriol. 144 577-591). The cells were plated on L-agar plates (Miller J. Experiments in Molecular Genetics, Cold Spring Harbour Laboratory, New York, p433) containing ampicillin to 100 µg/ml and incubated for 16 hours at 37°.

Clones were purified by streaking out samples on antibiotic supplemented L-agar plates and selecting single colonies. Recombinant clones (ie those resistant to tetracycline but sensitive to ampicillin) were subjected to an initial "colony hybridisation" screen (Grunstein M. and Hogness D. (1975) Proc. Nat. Acad. Sci. (USA) 72 3961-3965) using $^{32}$P labelled mRNA fragments. By comparing the hybridisation of individual cloned DNAs with a) mRNA from calf stomach, where chymosin specific sequences are found, and b) mRNA from calf livers, where there are no chymosin specific sequences, a number of putative chymosin specific clones were selected. A further colony hybridisation screen using cDNA extended from the specific primer described above confirmed the nature of the positive chymosin clones.

DNA derived from several such positive colonies exhibited restriction enzyme digestion patterns in accordance with the sequence elucidated for cDNA obtained from prochymosin mRNA by hybridisation priming (see above). The DNA from two such clones were each over half the length of the complete prochymosin gene. One section of DNA was the product of transcription from the 3' end of the prochymosin mRNA to a point before the 5' end of the prochymosin mRNA the transcription being oligo(dT)$_{12-18}$ primed. The other section of DNA was apparently obtained by transcription from a point after the 3' end of the prochymosin mRNA to the 5' end of the prochymosin mRNA.

The two clones containing these pieces of DNA were desiganted B18 and G1 respectively and a scale stricton site map of DNA from clones B18 and G1 is given in Figure 3(a). This diagram shows a number of restriction sites which exist in the overlapping portions of DNA derived from G1 and B18 which allow for ligation of the two partial prochymosin genes to produce a complete gene. In Figure 3(a) and 3(b) the restriction sites are denoted as follows:

Pst = P          Bam HI = B
KpnI = K         AvaII  = AV
SmaI = S         AluI   = A
RI   = R         BglII  = G

The recombinant clone designated 118 (Figure 3(b)) was constructed by joining the 5' portion of clone G1 and the 3' portion of clone A36 through their common RI site using standard restriction and ligation methods.

The techniques of Maxam and Gilbert (Proc. Nat. Acad. Sci. USA 74 560 (1977) have been used to elucidate the complete nucleotide sequence of the clone 118. In Figure 3 the arrows show the sites and directions from which DNA sequencing was carried out. The broken line arrow is the sequence obtained by extension of the synthetic primer. The result of this analysis is given in Figure 4 which shows the coding strand sequence and the corresponding amino acid sequence.

In general the nucleotide sequence is in accord with previously published amino acid sequences for this protein. However, the published sequences show aspartic acid in the sequence at amino acids 202 and 214 (indicated by asterisks in Figure 4). We have shown that these amino acids are in fact asparagine. The normal chemical techniques of amino acid sequencing readily confuse asparagine with aspartic acid, and by performing nucleotide sequencing this ambiguity has been removed. We have also found a short nucleotide sequence coding for an amino-terminal addendum to the prochymosin protein. This suggests that prochymosin is initially produced as a preprochymosin. Such a precursor of prochymosin has not previously been reported, but it is similar to the known leader sequences found on a variety of other secreted proteins (Davis B.D. and Tai P-C (1980) Nature 283 433-438) which are thought to be involved in the export of such proteins from cells.

The complete preprochymosin gene (or other DNA constructions derived from it) isolated from a recominbant clone may then be transferred into a vector designed to exhibit a satisfactory rate of expression of the relevant protein.

The translation of a structural gene into a protein requires inter alia the presence of a 'start' codon (ATG) as the codon coding for the first amino acid of the protein to be expressed. Expression of preprochymosin may be achieved directly since the amino acid sequence of the protein begins with a methionine residue, for which the codon is ATG. The expression of chymosin and prochymosin is not directly possible. Instead a fusion protein must be expressed. The N-terminal additional protein comprise any small protein having a methionine group as its first amino acid. In the following examples an ATG codon is attached to the gene for chymosin or prochymosin, the resulting expressed proteins being methionine-chymosin (met-chymosin) or methionine-prochymosin (met-prochymosin) respectively. We have prepared DNA constructions for the expression of met-chymosin, met-prochymosin or preprochymosin in E. coli and also in yeast (Saccharomyces cerevisiae).

A. Construction of expression plasmids for E. coli

1. Expression of methionine-prochymosin

The plasmid A36 (Fig. 3) was digested under standard conditions with the enzyme Pst I, to excise the cloned 900 base pair insert carrying the central portion of the prochymosin gene. The DNA was subjected to a very short (30 second) digest with the enzyme Bal 31 and the resulting blunt ended molecules and synthetic Hind III linker molecules (C.C.A.G.C.T.T.G.G) ligated to them under standard conditions using T4 DNA ligase. This material was digested with Hind III and then separated on a 5% acrylamide gel. The 900 bp fragment with Hind III cohesive termini was isolated from the gel by crushing and soaking in buffer followed by phenol extraction and ethanol precipitation. It was then recloned by standard methods into pAT 153 which had been cut with Hind III and dephosphorylated with bacterial alkaline phosphatase. This new plasmid was designated C5.

C5 DNA was digested with Hind III, dephosphorylated with alkaline phosphatase and digested with RI to yield a fragment (fragment I) having a 5'-phosphate at its RI cohesive end and a 5'-hydroxyl at its HIND III cohesive end.

The plasmid B31 (Fig. 3) was cut with Bam HI and to its cohesive ends were ligated two chemically synthesised oligonucleotides, one with a 5' hydroxyl terminus and the other with a 5'-phosphate terminus. These were synthesised by the methods referred to earlier for the synthesis of the synthetic cDNA primers.

OH - G.C.T.G.A.A.T.C.A.C.T.C.G.
C.G.A.C.T.T.T.A.G.T.G.A.G.C.C.T.A.G - OP

This ligation restores the missing sequence removed by the Bam HI digest which cuts at nucleotide 14 of the prochymosin gene (Fig. 4). The ligated DNA was cut with RI to produce a small fragment carrying the amino terminal region of prochymosin, having a blunt end with a 5'-hydroxyl group and an RI cohesive end with a 5'-phosphate (Fragment 2).

Fragment 1 (500 ng) and Fragment 2 (200 ng) were ligated together using T4 DNA ligase and the products resolved on a 5% acrylamide gel. The fragment corresponding to 1+2 was cut out and extracted from the gel as previously described.

This fragment was then cloned into a plasmid designated pCT54 (see restriction map - Fig. 5). This plasmid is derived from pAT 153 by cloning into it an E. Coli trp promoter/operator fragment and a phase T7 transcription terminator fragment using techniques well known to the art. It contains an initiation codon and a so-called "Shine and Delgarno sequence", important for ribosome binding. The nucleotide sequence of this plasmid in the area around the initiation site is given below:-

```
                              Cla I           Bcl I                      RI
                          ┌────────┐┌──────────────┐        ┌───────────────┐
5' - A.A.A.  A.A.G.G.  G.T.A.T.C.G.A.T.T.G.A.T.C.A. A.T.G.  A.A.T.T.C - 3'

     Shine and                                              Initiator
     Delgarno                                               codon
     sequence
```

The restriction enzyme sites between the SD sequence and the ATG allow for the adjustment of the ATG-SD distance which is an important parameter in maximising expression rates.  This plasmid also has  a Hind III restriction site just to the 3' side of the RI site.  pCT 54 was cut with RI and then digested with S1 nuclease to produce blunt ends using S1 nuclease.  10 g of DNA was digested with 300 units of S1 nuclease for 30 minutes at 20° in a buffer containing 0.3M NaCl, 0.025M NaAc pH 4.6 and 0.001M $ZnSO_4$.  The solution was deproteinised with phenol and the DNA recovered by ethanol precipitation.

The resulting blunt ended molecule was cut with Sa1 I to generate a short blunt end - Sal I fragment which was isolated from an acrylamide gel as described above. Further pCT 54 was cut with Hind III and Sal I to produce a large Hind III - Sal I fragment which was also isolated from an acrylamide gel.  The large Hind III - Sal I fragment (90 ng), the small blunt end - Sal I fragment (30 ng) and the reconstructed prochymosin fragment (1+2) were ligated together with T4 DNA ligase and the resulting DNA transformed into E. coli HB101 by standard procedures. Eight prochymosin clones were isolated and when the areas around the ATG codon were sequenced by the methods referred to previously they were all found to have the same nucleotide sequence, namely:-

```
                         Cla I              Bcl I
5' --- A.A.A. A.A.G.G. G.T.A.T.C.G.A.T.T.G.A.T.C.A. A.T.G. G.C.T.
promoter                                            met     ala

  .G.A.A.A.T.C.A.C.T. ........
    glu   ile   thr
```

The plasmids thus contain sequence coding for the complete amino acid sequence of prochymosin with an additional methionine residue at the amino terminus. The distance between the SD and ATG sequences is however too great for good expression. This distance was reduced by cutting the DNA at the Cla I site and creating blunt ended species by digestion with S1 nuclease as described above. This DNA, on religation with T4 DNA ligase and transformation into E. coli HB101, gave rise to a plasmid designated pCT 70 (see restriction map - Fig. 5). The DNA sequence of this plasmid in the region around the ATG is :-

prochymosin

Bcl I

--- A.A.G.G. G.T.A.T.T.T.G.A.T.C.A. A.T.G. G.C.T.G.A.A.A.T.C. ---

S.D.                        .met    ala   glu   ile

Expression of met. prochymosin from pCT 70 is described below.


2.    Expression of methionine-chymosin

Construction of chymosin expression vector commenced from pCT 70 the prochymosin plasmid described above.  pCT 70 DNA was digested with Bcl I under conditions where cutting of the DNA was not complete and full length linear molecules were the main product.  These could have been cut at either of the Bcl I sites in pCT 70.  Digestion of the DNA with RI followed by selection and isolation of the largest fragment ensured that the species taken extended from the RI site in the met-prochymosin gene round to the Bcl I site nearest to the start of the gene. The small fragment needed to complete the met-chymosin gene was derived from a plasmid pCT 57 (Fig. 5) which is derived from pCT 54 by inserting the prochymosin gene (from the Bam site at position 14 to the 3' end) into the RI site using a chemically synthesised linker:-

(5') A.A.T.T.C.T.A.T.C.A.C.T.C.G.

G.A.T.A.G.T.G.A.G.C.C.T.A.G. (5')

which gives a sequence of 3 amino acids at the N-terminus which differ from the natural amino acid sequence (i.e. is a pseudo prochymosin).  pCT 57 plasmid DNA was restricted with Bal I and a chemically synthesised linker molecule:-

OH - G.A.T.C.A.A.T.G.G.G.T.G.A.A.G.T.A.G.

T.T.A.C.C.C.A.C.T.T.C.A.T.C.   -OP

was ligated to the blunt Bal I ends of the fragments with T4 DNA ligase.  The 5' - G.A.T.C ends of the new fragments can be ligated to cohesive ends from Bam HI or Bcl I or Bgl II digests.  The DNA was further restricted with RI to generate a fragment:-

```
                    met     gly     glu     val

HO - G.A.T.C.A. A.T.G. G.G.T.G.A.A.G.T.A.G.        Bgl II        R I

        T.T.A.C. C.C.A.C.T.T.C.A.T.C
```